## Europäisches Patentamt

## European Patent Office

## Office européen des brevets

(19)

(11) Veröffentlichungsnummer: **0 056 869**
**B1**

(12) **EUROPÄISCHE PATENTSCHRIFT**

(45) Veröffentlichungstag der Patentschrift:
25.01.84

(51) Int. Cl.³: **B 01 J 23/96, C 07 C 67/00**

(21) Anmeldenummer: **81110728.3**

(22) Anmeldetag: **23.12.81**

(54) **Verfahren zur Regenerierung von Palladium und/oder Platin und Tellur enthaltenden Trägerkatalysatoren.**

(30) Priorität: **23.01.81 DE 3102087**

(43) Veröffentlichungstag der Anmeldung:
**04.08.82 Patentblatt 82/31**

(45) Bekanntmachung des Hinweises auf die Patenterteilung:
**25.01.84 Patentblatt 84/4**

(84) Benannte Vertragsstaaten:
**BE DE FR GB IT NL**

(56) Entgegenhaltungen:
**EP - A - 0 005 467**
**EP - A - 0 027 979**
**DE - A - 2 444 004**
**NL - A - 6 409 545**

(73) Patentinhaber: **BASF Aktiengesellschaft,**
**Carl-Bosch-Strasse 38, D-6700 Ludwigshafen (DE)**

(72) Erfinder: **Schnabel, Rolf, Dr., Tilsiter Strasse 9,**
**D-6707 Schifferstadt (DE)**
Erfinder: **Weitz, Hans-Martin, Dr., Auf dem Koeppel 40,**
**D-6702 Bad Duerkheim (DE)**
Erfinder: **Fischer, Rolf, Dr., Bergstrasse 98,**
**D-6900 Heidelberg (DE)**

## Verfahren zur Regenerierung von Palladium und/oder Platin und Tellur enthaltenden Trägerkatalysatoren

Diese Erfindung betrifft ein Verfahren zur Regenerierung von Palladium und/oder Platin und Tellur enthaltenden Trägerkatalysatoren, die für Acyloxilierungen verwendet werden.

Katalysatoren, die für Acyloxilierungen verwendet werden, sind z.B. Palladium und/oder Platin und weiterhin Tellur und gegebenenfalls Kupfer enthaltende Trägerkatalysatoren. Sie werden z.B. für die Herstellung von Butendiolestern durch Umsetzung von Butadienen mit niederen Carbonsäuren und Sauerstoff herangezogen. Butendiolester, wie 1,4-Diacetoxy-2-buten, sind wertvolle Zwischenprodukte, z.B. für die Herstellung von Butendiol-1,4, Butandiol-1,4 und Tetrahydrofuran. 1-Butendiol-3,4-diacetat (Vinylglykoldiacetat) ist als Zwischenprodukt zur Herstellung von Vitaminen und anderen biologisch wirksamen Verbindungen geeignet und 2-Methyl-1,4-diacetoxy-2-butene oder 1,1,4-Triacetoxy-2-methyl-2-butene sind wertvolle Zwischenprodukte, z.B. für die Synthese von Terpenverbindungen.

Zu den wesentlichen Qualitätsmerkmalen von Katalysatoren zählt die Katalysatoraktivität, d.h. die unter definierten Reaktionsbedingungen pro Katalysatormengeneinheit und pro Zeiteinheit hergestellte Menge an Wertprodukt. Im Falle der kontinuierlich durchgeführten Acetoxilierung von Butadien, bei der man Butadien mit Essigsäure und Sauerstoff umsetzt, ist das die g-Menge an Butendioldiacetaten pro kg Katalysator und Stunde. Eine weitere wichtige Grösse stellt die Katalysatorstandzeit dar, das ist der Zeitraum, in dem der Katalysator eine hinreichend hohe Aktivität besitzt. Trägt man die Katalysatoraktivität gegen die Katalysatorstandzeit auf, so erhält man eine Kurve, deren bestimmtes Integral die Gesamtmenge an produziertem Wertprodukt pro Katalysatormenge angibt. Auch dieser Wert (Produktivität) kann als Qualitätskriterium für den verwendeten Katalysator herangezogen werden. Es muss beachtet werden, dass für eine technische Verwendung eine zu kurze Standzeit (bei hoher Katalysatoraktivität) die Anforderungen ebenso wenig erfüllt, wie eine zu niedrige Katalysatoraktivität über sehr lange Standzeiten, auch wenn in diesen beiden Extremfällen die Produktivität an sich sehr gut ist.

Die genannten Qualitätsmerkmale des Katalysators werden durch den wirtschaftlichen Aufwand bei der Beschaffung der Ausgangsstoffe und der Herstellung des Katalysators relativiert. Ein teurer Katalysator muss, um die gleiche Rentabilität wie ein billiger Katalysator zu erreichen, z.B. eine entsprechend höhere Produktivität aufweisen.

Bei der Anwendung von Katalysatoren mit hohen Edelmetallgehalten wird man im allgemeinen nicht ohne eine Reaktivierung oder Aufarbeitung der Katalysatoren auskommen, da die Aktivität jedes Katalysators nach unterschiedlichen Standzeiten unter einen wirtschaftlich tragbaren Wert abfällt.

Eine möglichst wirtschaftliche Regenerierung bzw. Aufarbeitung des desaktivierten Katalysators ist daher wünschenswert. So sind für eine Regenerierung von Palladium und weitere Metallkomponenten enthaltenden Trägerkatalysatoren schon mehrere Vorschläge gemacht worden. Beispielsweise ist aus der japanischen Auslegeschrift 9993 (1979) bekannt, dass sich Acetoxilierungskatalysatoren, die Palladium und Tellur auf Aktivkohle als Träger enthalten, dadurch reaktivieren lassen, dass man die Katalysatoren verminderter Aktivität bei 300 bis 600°C mit Wasserstoff behandelt. Eine ähnliche Wirkung erzielt man, wenn man einen derartigen Katalysator verminderter Aktivität zunächst bei 300 bis 600°C mit Wasserstoff, dann bei 150 bis 350°C mit Sauerstoff und zuletzt wiederum bei 300 bis 600°C mit Wasserstoff behandelt [japanische Auslegeschrift 9992 (1979)]. Es ist weiterhin bekannt, Katalysatoren mit Palladium, Rhodium, Ruthenium oder Platin auf Aktivkohle als Träger, die zusätzlich Selen und/oder Tellur enthalten können, dadurch zu reaktivieren, dass man sie zunächst mit einer Mineralsäure wie Flussäure, Salzsäure oder Salpetersäure behandelt und anschliessend bei 200 bis 500° reduziert [Japan Kokai 39 283 (1978)]. Weiterhin wurde eine Regenerierung von palladiumhaltigen Aktivkohle-Trägerkatalysatoren, die zusätzlich Selen, Bismut und/oder Tellur enthalten können, durch Behandlung mit Essigsäure und Sauerstoff durchgeführt.

Nach diesen Methoden lässt sich zwar die ursprüngliche Katalysatoraktivität wiederherstellen. Bei kontinuierlicher Fahrweise zeigt sich jedoch, dass die so reaktivierten Katalysatoren nur relativ kurze Standzeiten bei relativ niedrigen Aktivitäten und somit niedrige Produktivitäten aufweisen. Ferner ist von Nachteil, dass die als Trägermaterial verwendete Aktivkohle bei der Katalysatorreaktivierung durch Oxidationsmittel, wie Sauerstoff oder Salpetersäure angegriffen wird. Wegen des hohen Palladiumpreises ist man andererseits gezwungen, das Palladium von nach langen Reaktionszeiten nicht mehr reaktivierbaren Katalysatorträgern abzulösen und wiederzugewinnen. Auch hierzu ist Salpetersäure erforderlich, die nullwertiges Palladium zu zweiwertigem Palladium unter Stickoxidentwicklung oxidiert. Es ist aber bekannt, dass bei der Behandlung von Aktivkohle mit Salpetersäure explosionsartige Verpuffungen auftreten können, die ein sicheres Arbeiten wesentlich erschweren.

Es wurde nun gefunden, dass sich Palladium und/oder Platin und Tellur und gegebenenfalls Kupfer enthaltende Trägerkatalysatoren für Acyloxilierungen mit einem Kupfergehalt von A = 0 bis 2, wobei A die Grammatome Kupfer, bezogen auf 1 Grammatom des Palladiums und/oder Platins bedeutet, besonders vorteilhaft dadurch regenerieren lassen, dass man auf die zu regenerierenden Katalysatoren soviel Kupfer aufbringt, dass die regenerierten Katalysatoren einen Kupfergehalt von B = A + C aufweisen, wobei A die obengenannte Bedeutung hat und C eine Zahl von 0,5 bis 6, insbesondere 1 bis 4, bedeutet.

Die zu regenerierenden Acyloxilierungskatalysatoren werden z.B. in der DE-PS 22 17 452, der DE-OS 29 43 407 und der DE-OS 28 20 519 beschrieben. Katalysatoren der genannten Art enthalten beispielsweise, bezogen auf das Katalysatorgewicht, 0,1 bis 25 Gew.% Palladium oder Platin, 0,01 bis 30 Gew.% Tellur und 0 bis 30 Gew.% Kupfer. Von den kupferhaltigen Katalysatoren sind solche bevorzugt, die je Grammatom Palladium oder Platin 0,01 bis 6, vorzugsweise 1 bis 3,5 Grammatome Kupfer und 0,01 bis 1, vorzugsweise 0,01 bis 0,4 Grammatome Tellur enthalten und in denen intermetallische Phasen der Zusammensetzung $PdCu_3$, $PtCu_3$ oder PdCu, PtCu röntgenographisch nachweisbar sind.

Die Gesamtmenge der auf den Träger aufgebrachten katalytisch wirksamen Metalle beträgt bei diesen Katalysatoren, bezogen auf den Katalysatorträger, z.B. 0,01 bis 30 Gew.%. Als Trägermaterial enthalten die Katalysatoren z.B. Aktivkohle, Bauxit, Bimsstein, Kieselgel, Kieselgur oder andere Formen der Kieselsäure, Magnesia, Ton oder Tonerde.

Die genannten Katalysatoren werden für die Herstellung von Butendiolestern durch Acyloxilierung von Butadien oder substituierten 1,3-Dienen, wie Isopren, 2,3-Dimethyl-1,3-butadien, 1,3-Pentadienen, wie Piperylen oder durch Acyloxygruppen substituierten 1,3-Butadienen, wie 1-Acetoxy-1,3-butadien, 1-Acetoxy-2-methyl-1,3-butadien oder 1-Acetoxy-3-methyl-1,3-butadien verwendet. Dabei werden die genannten Diolefine für sich oder als Mischungen, die z.B. auch noch andere Kohlenwasserstoffe, wie Monoolefine und Paraffinkohlenwasserstoffe enthalten, eingesetzt. Solche Mischungen stehen z.B. als sogenannte $C_4$-Schnitte zur Verfügung. Die Acyloxilierung wird bekanntlich so durchgeführt, dass man die Diene in Gegenwart der Katalysatoren in der Gas- oder Flüssigphase bei Temperaturen von 70 bis 180°C mit Sauerstoff und niedermolekularen Carbonsäuren, wie Ameisensäure, Essigsäure oder Propionsäure behandelt. Der Reaktionsdruck ist durch die Verfahrensweise gegeben und kann zwischen atmospärischem Druck und z.B. 100 bar betragen. Die Acyloxilierung wird chargenweise oder kontinuierlich z.B. mit fixiertem Bett, Wirbelbett oder Dreiphasenfliessbett durchgeführt.

Die erfindungsgemässe Regenerierung kann beispielsweise folgendermassen durchgeführt werden: Zweckmässigerweise wird der zu regenerierende Katalysator vor dem erfindungsgemässen Aufbringen von Kupfer zunächst mit einem Lösungsmittel behandelt. Als Lösungsmittel sind z.B. die Carbonsäuren geeignet, mit denen die vorangegangene Acyloxilierung durchgeführt wurde. Essigsäure ist besonders gut geeignet. Man behandelt den zu regenerierenden Katalysator mit dem Lösungsmittel z.B. 0,5 bis 12 Stunden bei Temperaturen von 20 bis 200°C, wobei man z.B. 5 bis 50 Gewichtsteile Lösungsmittel je 1 Gewichtsteil Katalysator anwendet. Anstelle von Carbonsäuren können auch andere Lösungsmittel, wie Ester, z.B. Essigsäureethylester, Alkohole z.B. Methanol, Ethanol oder Kohlenwasserstoffe, z.B. Pentane, Hexane, Benzol oder Toluol, verwendet werden.

Nach dieser Vorbehandlung wird der Katalysator zur Entfernung von Lösungsmittel getrocknet. Man trocknet z.B. in einem Inertgasstrom bei 50 bis 200°C unter Normaldruck oder im Vakuum. Zum Aufbringen von Kupfer wird der zu reaktivierende Katalysator beispielsweise zunächst mit einer Kupfersalzlösung getränkt. Die hierfür verwendete Kupferverbindung, die ein- oder zweiwertig sein kann, ist nicht entscheidend. So kann man z.B. Kupfersulfat, Kupferchlorid, Kupfernitrat, Kupferacetat oder Kupfer enthaltende Komplexverbindungen verwenden. Es ist auch möglich, von metallischem Kupfer oder von Kupferoxiden oder Hydroxiden auszugehen, diese in Säuren zu lösen und die Kupfersalzlösungen zu verwenden. Als Lösungsmittel für Kupfersalze kommen beispielsweise Wasser, Mineralsäuren, wie Salzsäure, Salpetersäure oder, soweit die Löslichkeit der Salze nicht zu gering ist, organische Lösungsmittel, wie Alkohole, Ether oder Carbonsäureester in Betracht. Lässt man Kupfersalze, gelöst in Säuren, wie Salpetersäure, auf den zu regenerierenden Katalysator einwirken, so kann ein Teil der Metalle vom Katalysatorträger abgelöst werden und in die Kupfersalzlösung gelangen. Dies ist nicht störend, da alle diese Metallsalze wieder auf dem Träger abgeschieden werden. Es ist natürlich prinzipiell auch möglich, das gesamte Palladium und/oder Platin, Tellur und/oder Kupfer zunächst vom Träger abzulösen, die entstehende Metallsalzlösung mit der Kupfersalzlösung zu vereinigen und die Salze durch Abziehen des Lösungsmittels wieder auf dem, gegebenenfalls neuen oder in seiner Menge ergänzten Katalysatorträger niederzuschlagen.

Es ist weiterhin auch möglich, metallisches Kupfer im Vakuum auf den zu regenerierenden Katalysator aufzudampfen.

Die aufzubringende Kupfermenge wird so gewählt, dass der Katalysator nach der Regenerierung pro Grammatom Palladium und/oder Platin 0,5 bis 6, insbesondere 2 bis 4 g-Äquivalente Kupfer enthält oder dass der zu regenerierende Katalysator, der bereits Kupfer enthält, nach der Regenerierung pro g-Atom Palladium und/oder Platin einen um 0,5 bis 6 g-Atome Kupfer erhöhten Kupfergehalt aufweist. Es ist möglich, aber aus wirtschaftlichen Gründen nicht sinnvoll, noch grössere Cu-Mengen aufzubringen. Wird ein Katalysator regeneriert, der ursprünglich schon kupferhaltig war, so wird man zweckmässigerweise die bei der vorangegangenen Acyloxilierung vom Katalysator abgelöste Kupfermenge ergänzen. Es ist auch möglich, gleichzeitig mit dem Aufbringen von Kupfer den Gehalt an Palladium, Platin und/oder Tellur durch Zugabe entsprechender Verbindungen zu ergänzen, obwohl im allgemeinen nur geringfügige Verluste an diesen Metallen beobachtet werden. Auch Katalysatorträger können gegebenenfalls ergänzt werden. Palladium und/oder Platin, Tellur und/oder Kupfer können gleichzeitig oder in beliebiger Reihenfolge nacheinander auf dem Träger abgeschieden werden.

Die so erhaltenen, mit Metallsalzlösungen getränkten Katalysatoren werden dann auf an sich bekannte Weise fertiggestellt. Zunächst werden sie zur Entfernung der Lösungsmittel bei Temperaturen von 50 bis 200°C unter Normaldruck oder im Vakuum getrocknet. Anschliessend werden die so erhaltenen rohen Katalysatoren in einem reduzierend wirkenden Gasstrom auf höhere Temperaturen erhitzt. Man kann jedes Reduktionsverfahren anwenden, durch welches die verwendeten Elemente in den metallischen Zustand überführt werden. Als Reduktionsmittel sind beispielsweise Wasserstoff, Methanol, Hydrazin oder Formaldehyd geeignet. Die Salze können z.B. in einem Gasstrom aus Wasserstoff oder Stickstoff, der mit einer reduzierenden Verbindung, wie Hydrazin, Methanol oder Formaldehyd beladen ist, reduziert werden. Die Reduktion des getrockneten Katalysators kann auch mit flüssigen Reduktionsmitteln geschehen. Die Reduktionstemperatur kann beispielsweise 100 bis 500°C, insbesondere 200°C bis 400°C betragen. Erforderlich ist, dass die Metalle nicht in Form zufälliger physikalischer Mischungen vorliegen, sondern, dass der Katalysator die Metalle Palladium, Platin und Kupfer in einer intermetallischen Palladium-Kupfer- oder Platin-Kupfer-Verbindung enthält. Um dies so zu erreichen, verfährt man z.B. wie in der DT-OS 28 20 519 angegeben, indem man die Katalysatoren auf 400 bis 900°C, vorzugsweise 500 bis 800°C erhitzt. Im allgemeinen steht dabei die Dauer des Erhitzens und die Höhe der erreichten Temperatur in einem gewissen Zusammenhang. Es hat sich als zweckmässig erwiesen, z.B. 15 Minuten bis 4 Stunden auf 400 bis 900°C zu erhitzen, wobei im allgemeinen die kürzere Erhitzungszeit der höheren Temperatur zugeordnet ist. Da sich beim Erhitzen schliesslich eine gewisse Stabilisierung des einmal erreichten katalytisch wirksamen Zustandes einstellt, ist die Anwendung einer bestimmten Höchstzeit im allgemeinen nicht erforderlich. Falls der Träger oder die Metalle unter den Bedingungen des Erhitzens zur Oxidation neigen, wird man zweckmässig das Erhitzen in Gegenwart einer reduzierenden Atmosphäre, z.B. in reinem Wasserstoff oder eines Inertgases wie Stickstoff vornehmen. In dem so behandelten Katalysator lässt sich das Vorliegen der intermetallischen Palladium-Kupfer- oder Platin-Kupfer-Verbindung durch eine Röntgenstrukturanalyse leicht feststellen.

Verfahren werden die Palladium und/oder Platin und Tellur und gegebenenfalls Kupfer enthaltenden Trägerkatalysatoren auf einfache Weise besonders wirksam und nachhaltig regeneriert. Dieses vorteilhafte Ergebnis ist u.a. deshalb überraschend, da beim erneuten Einsatz der regenerierten Katalysatoren für die Acetoxilierung von Butadien die hohe Aktivität des Katalysators lange erhalten bleibt, obwohl ein beträchtlicher Teil des bei der Regenerierung aufgebrachten Kupfers schon nach kurzen Betriebszeiten wieder abgelöst wird. Dies ist besonders deutlich aus dem Diagramm zum Beispiel 4 zu ersehen. Überraschend ist auch, dass bei Trägerkatalysatoren mit anorganischem Trägermaterial, wie Silikagel im Vergleich mit solchen, die Aktivkohle als Träger enthalten, noch günstigere Regenerierungseffekte der geschilderten Art erzielt werden. Darüber hinaus muss es als sehr überraschend bezeichnet werden, dass man mit Katalysatoren, die anorganische Träger, wie Silikagel enthalten, nach ihrer Regenerierung sogar höhere Butendioldiacetat-Mengen und längere Katalysatorstandzeiten erzielt als sie mit den ursprünglichen Katalysatoren erhalten werden konnten.

Beispiel 1

a) Verwendung eines Pd, Cu und Te enthaltenden Trägerkatalysators für die Acetoxilierung von Butadien:

5,2 kg eines Katalysators, der 4,6 Gew.% Pd, 7,5 Gew.% Cu und 0,4 Gew.% Te auf 4 mm-Aktivkohlesträngen enthält (2,73 Grammatome Kupfer, bezogen auf 1 Grammatom Palladium), werden in ein beheizbares Reaktionsrohr von 45 mm Innendurchmesser und 7 m Höhe eingefüllt. In Festbett-Rieselfahrweise werden bei 90 bar und 95°C stündlich 20 Nm³ Stickstoff, 0,4–1 Nm³ Sauerstoff, 10 l Essigsäure und 1,5 l Butadien von oben durch die Katalysatorschüttung geleitet.

Die Reaktionsausträge werden in diesen und den folgenden Beispielen gaschromatographisch auf ihren Gehalt an den Wertprodukten trans-1,4-Diacetoxy-2-buten (trans-1,4-BEDA), cis-1,4-Diacetoxy-2-buten (cis-1,4-BEDA) und 3,4-Diacetoxy-1-buten (3,4-BEDA) untersucht. Bei der im folgenden angegebenen Bezeichnung BEDA handelt es sich um die Summe der drei genannten isomeren Diacetoxybutene.

Die Aktivität beträgt am Anfang der Umsetzung 290 g BEDA/kg Katalysator·h. Nach einer Betriebszeit von 820 h ist die Aktivität auf 61 g BEDA/kg Katalysator·h abgefallen. Der Metallgehalt des Katalysators beträgt jetzt 5,5 Gew.% Pd, 2,4 Gew.% Cu und 0,3 Gew.% Te. Dieser teilweise desaktivierte Katalysator enthält somit je Grammatom Pd 0,73 Grammatom Cu.

b) Regenerierung und Wiederverwendung des Katalysators:

Der desaktivierte Katalysator gemäss Absatz a) wird mit einer Lösung von 400 g Cu in 6,8 l 3n Salpetersäure getränkt. Man verwendet gerade so viel Lösung, dass eine gleichmässige Flüssigkeitsbenetzung des Katalysators gewährleistet ist. Nach ½stündiger Durchmischung der flüssigen und der festen Phase wird bei vermindertem Druck (Wasserstrahlvakuum) die Flüssigphase bei 85 bis 90°C abgedampft, bis sich kein Kondensat mehr am Kühler bildet. Der Katalysator wird dann in ein beheizbares Rohr gefüllt und unter Normaldruck im Stickstoffstrom langsam auf 150°C aufgeheizt und so lange auf dieser Temperatur gehalten, bis kein Kondensat und keine nitrosen Gase mehr ausgetragen werden. Dann wird der Stickstoffstrom bei Raumtemperatur mit Methanol gesättigt und 2 Tage lang durch die auf 250°C bzw. 400°C thermostatierte Katalysatorschüttung mit 3 Nl/cm$^2$ · h geleitet. Schliesslich wird der Katalysator auf 800°C aufgeheizt und ½ Stunde bei dieser Temperatur gehalten, wobei Wasserstoff mit 3 Nl/cm$^2$ · h durch die Katalysatorschüttung geleitet wird. Die Abkühlung auf Raumtemperatur erfolgt unter einem Inertgasstrom, z.B. Stickstoff.

Der auf diese Weise regenerierte Katalysator enthält 4,6 Gew.% Pd, 5,6 Gew.% Cu und 0,6 Gew.% Te. Er enthält somit je Grammatom Pd 2,05 Grammatome Cu.

Der regenerierte Katalysator wird wie in Absatz a) beschrieben verwendet. Seine Anfangsaktivität beträgt 440 g BEDA/kg Katalysator · h. Nach gleicher Betriebszeit von 820 h beträgt die Aktivität 62 g BEDA/kg Katalysator · h.

Beispiel 2

a) Verwendung eines Pd, Cu und Te enthaltenden Katalysators für die Acetoxilierung von Butadien:

800 g eines Katalysators, der 5,2 Gew.% Pd, 8,3 Gew.% Cu und 0,5 Gew.% Te auf Aktivkohle (0,8 bis 1,2 mm Split) enthält, (2,7 Grammatome Kupfer, bezogen auf 1 Grammatom Pd) wird in einem beheizbaren Reaktionsrohr von 45 mm Innendurchmesser und 4 m Höhe in einer Festbett-Sumpffahrweise getestet. Dazu werden unter einem Druck von 75 bar bei 95°C stündlich 25 Nl Sauerstoff, gelöst in 5 l Essigsäure, und 1,25 l C$_4$-Crackschnitt, der 25% Butadien enthält, von unten durch die Katalysatorschüttung gepumpt. Die Anfangsaktivität beträgt 410 g BEDA/kg Katalysator · h. Sie ist nach 400 h auf 274 g BEDA/kg Katalysator · h abgefallen. Der Metallgehalt des Katalysators beträgt jetzt 4,9 Gew.% Pd, 4,4 Gew.% Cu und 0,5 Gew.% Te. Der partiell desaktivierte Katalysator enthält somit je Grammatom Pd 1,5 Grammatome Cu.

b) Regenerierung und Wiederverwendung des Katalysators:

Der gemäss Absatz a) eingesetzte Katalysator wird mit einer Lösung von 41 g Cu in 300 ml 5n Salpetersäure getränkt und wie in Beispiel 1 beschrieben regeneriert. Seine Metallgehalte betragen jetzt 5,0 Gew.% Pd, 10,5 Gew.% Cu und 0,6 Gew.% Te. Je Grammatom Pd enthält der regenerierte Katalysator somit 3,5 Grammatome Cu.

Der regenerierte Katalysator wird, wie im Absatz a) dieses Beispiels beschrieben, zur Acetoxilierung eingesetzt. Seine Anfangsaktivität beträgt 525 g BEDA/kg Katalysator · h. Nach gleicher Betriebszeit von 400 h beträgt die Aktivität 275 g BEDA/kg Katalysator · h.

Beispiel 3

a) Verwendung eines Pd, Cu und Te enthaltenden Katalysators für die Acetoxilierung von Butadien:

3 g des ursprünglichen Katalysators von Beispiel 2 werden in einem Autoklaven von 50 ml Inhalt in 30 ml Reaktionsmedium mit einem Magnetrührstäbchen mit 150 U/min bei 20 bar und 95°C gerührt. Es werden stündlich 3 Nl Sauerstoff und 100 ml einer Lösung von 10 Gew.% Butadien in Essigsäure zugeführt; die entsprechende Menge Reaktionsmedium wird ausgetragen.

Die Anfangsaktivität des Katalysators beträgt 2300 g BEDA/kg Katalysator · h. Nach 250 h Versuchsdauer ist die Aktivität auf 370 g BEDA/kg Katalysator · h und der Cu-Gehalt im Katalysator um 60% zurückgegangen. Der desaktivierte Katalysator enthält je Grammatom Pd 1,07 Grammatome Cu.

b) Regenerierung und Wiederverwendung des Katalysators:

Entsprechend Beispiel 1 b) wird die Regenerierung so durchgeführt, dass sich der Cu-Gehalt auf 10 Gew.% erhöht (3,2 Grammatome Cu je Grammatom Pd). Beim erneuten Einsatz des regenerierten Katalysators gemäss Absatz a) beträgt die Aktivität am Anfang 2600 g BEDA/kg Katalysator · h und nach 250 h 340 g BEDA/kg Katalysator · h.

Beispiel 4

a) Verwendung eines Pd, Cu und Te enthaltenden Trägerkatalysators für die Acetoxylierung von Butadien:

4,3 kg eines Katalysators, der 4,7 Gew.% Pd, 7,8 Gew.% Cu (das sind 2,8 Grammatome Cu je Grammatom Pd) und 0,5 Gew.% Te auf SiO$_2$-Gel (1,2 bis 3,0 mm Split) enthält, werden wie in Beispiel 1 a) beschrieben verwendet. Die Anfangsaktivität beträgt 350 g BEDA/kg Katalysator · h. Nach einer Betriebszeit von 360 h ist die Aktivität auf 10% der Anfangsaktivität abgefallen. Der Metallgehalt wird am desaktivierten Katalysator zu 4,4 Gew.% Pd, 4,5 Gew.% Cu und 0,4 Gew.% Te gemessen (1,7 Grammatome Cu je Grammatom Pd).

b) Regenerierung des Katalysators:

Der desaktivierte Katalysator wird mit einer Lösung von 200 g Cu in 4,6 l 3n Salpetersäure getränkt und (wie in Beispiel 1 b) beschrieben) regeneriert. Seine Metallgehalte betragen danach 3,9 Gew.% Pd, 8,4 Gew.% Cu (= 3,6 Grammatome je Grammatom Pd) und 0,4 Gew.% Te. Verwendet man den regenerierten Katalysator entsprechend Beispiel 1 b), so beträgt die Anfangsaktivität 440 g BEDA/kg Katalysator · h. Nach weiteren 3700 h beträgt die Aktivität noch 382 g BEDA/kg Katalysator · h.

In dem Diagramm sind die Aktivität des Katalysators (–o–o–) und der Cu-Gehalt des desaktivierten Katalysators (–.–.–), weiterhin die Katalysatoraktivität und der Cu-Gehalt des reaktivierten Katalysators (Cu in Gew.%) als Funktion der Versuchsdauer (h = Stunden) dargestellt.

**Beispiel 5**

Der nach Beispiel 4 regenerierte Katalysator wird der im Beispiel 3 beschriebenen Acetoxilierung im Autoklaven unterzogen. Seine Anfangsaktivität beträgt 3330 g BEDA/kg Katalysator · h. Nach einer Betriebszeit von 250 h beträgt die Aktivität noch 1600 g BEDA/kg Katalysator · h.

**Beispiel 6**

a) Verwendung eines Pd, Cu und Te enthaltenden Trägerkatalysators für die Acetoxilierung von Butadien:

7,5 ml eines Katalysators, der 4,3 Gew.% Pd, 9,0 Gew.% Cu und 1,0 Gew.% Te auf $SiO_2$-Gel (0,8 bis 1,0 mm Split) enthält (er enthält somit 3,5 Grammatome Kupfer je 1 Grammatom Pd) werden in ein Rohr mit einem Inhalt von 125 ml eingefüllt. Das Rohr, das unten mit einem Sieb abgeschlossen ist und oben konusförmig in ein Rohr mit doppeltem Querschnitt übergeht, wird senkrecht in einem 1,5 l-Autoklaven angebracht. Bei 20 bar und 95°C werden dem Autoklaven stündlich 4 Nl Sauerstoff, 1 l Essigsäure und 0,1 l Butadien zugeführt. Gleichzeitig wird die entsprechende Menge des Reaktionsmediums ausgetragen. Das Reaktionsmedium wird mittels eines Propellers von unten nach oben so durch das Rohr geleitet, dass sich der Katalysator im Rohr von 125 ml Inhalt wirbelnd auf das Rohrvolumen verteilt.

Die Anfangsaktivität beträgt 3330 g BEDA/kg Katalysator · h. Nach einer Betriebszeit von 260 h beträgt die Aktivität 2000 g BEDA/kg Katalysator · h. Nach insgesamt 800 h ist die Aktivität auf 30% der Anfangskapazität abgesunken. Der Metallgehalt des Katalysators beträgt jetzt 4,3 Gew.% Pd, 2,8 Gew.% Cu und 1,0 Gew.% Te (1,1 Grammatome Cu je 1 Grammatom Pd).

b) Regenerierung und Wiederverwendung des Katalysators:

6,5 g des partiell desaktivierten Katalysators werden mit einer Lösung von 0,52 g Cu in 7,5 ml 3n Salpetersäure getränkt und wie im Beispiel 1 b) beschrieben regeneriert. Danach betragen die Metallgehalte 4,0 Gew.% Pd, 9,6 Gew.% Cu (4,0 Grammatome Cu je Grammatom Pd) und 1,0 Gew.% Te. Die Anfangsaktivität dieses Katalysators beträgt unter den in Absatz a) genannten Bedingungen 5150 g BEDA/kg Katalysator · h. Nach 260 h beträgt die Aktivität 3080 g BEDA/kg Katalysator · h.

**Beispiel 7**

a) Verwendung eines Pd und Te enthaltenden Trägerkatalysators für die Acetoxylierung von Butadien:

3 g eines Katalysators, der 6,2 Gew.% Pd und 1,1 Gew.% Te auf Aktivkohle enthält, wird wie im Beispiel 3, Absatz a) beschrieben verwendet. Er hat dabei eine Anfangsaktivität von 1600 g BEDA/kg Katalysator · h. Nach einer Betriebszeit von 250 h beträgt die Aktivität 400 g BEDA/kg Katalysator · h.

b) Regenerierung und Wiederverwendung des Katalysators:

Entsprechend Beispiel 3 b) wird die Regenerierung so durchgeführt, dass man einen Katalysator mit 11,2 Gew.% Cu erhält (3,0 Grammatome Cu je Grammatom Pd). Beim erneuten Einsatz des regenerierten Katalysators gemäss Absatz a) beträgt die Aktivität am Anfang 5000 g BEDA/kg Katalysator · h und nach 250 h 1930 g BEDA/kg Katalysator · h.

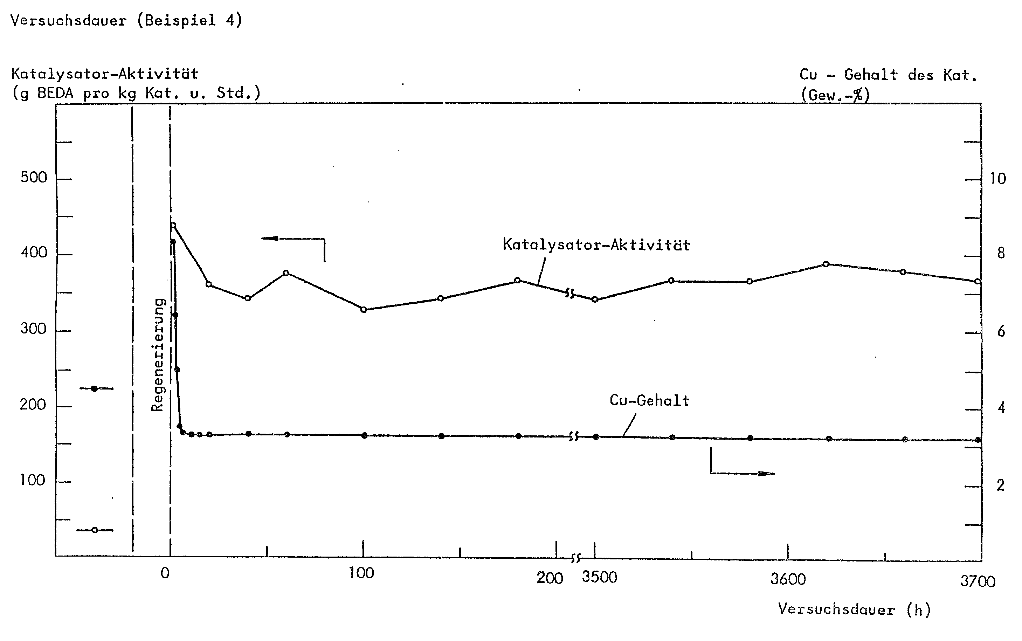

**Patentansprüche**

1. Verfahren zur Regenerierung von Palladium und/oder Platin und Tellur und gegebenenfalls Kupfer enthaltenden Trägerkatalysatoren für Acyloxilierungen mit einem Kupfergehalt von A = 0 bis 2, wobei A die Grammatome Kupfer, bezogen auf 1 Grammatom des Palladiums und/oder Platins bedeutet, dadurch gekennzeichnet, dass man auf die zu regenerierenden Katalysatoren soviel Kupfer aufbringt, dass die regenerierten Katalysatoren einen Kupfergehalt von B = A + C aufweisen, wobei A die obengenannte Bedeutung hat und C eine Zahl von 0,5 bis 6 bedeutet.

2. Verfahren nach Anspruch 1, dadurch gekennzeichnet, dass man Trägerkatalysatoren reaktiviert, die 0,1 bis 25 Gewichtsprozent Palladium oder Platin, 0,01 bis 30 Gewichtsprozent Tellur und 0 bis 30 Gewichtsprozent Kupfer enthalten.

3. Verfahren nach Anspruch 1, dadurch gekennzeichnet, dass man Trägerkatalysatoren reaktiviert, die je Grammatom Palladium oder Platin 0,01 bis 2 Grammatome Kupfer und 0,01 bis 1 Grammatom Tellur enthalten.

4. Verfahren nach Anspruch 1, dadurch gekennzeichnet, dass die zu reaktivierenden Trägerkatalysatoren als Trägermaterial Aktivkohle enthalten.

5. Verfahren nach Anspruch 1, dadurch gekennzeichnet, dass die zu reaktivierenden Trägerkatalysatoren als Trägermaterial Silikagel enthalten.

6. Verfahren nach Anspruch 1, dadurch gekennzeichnet, dass man die Trägerkatalysatoren nach dem Aufbringen des Kupfers auf 400 bis 900°C erhitzt.

**Revendications**

1. Procédé pour régénérer des catalyseurs au palladium et (ou) au platine et au tellure sur support pour les réactions d'acyloxylations, pouvant le cas échéant contenir aussi du cuivre avec une teneur en cuivre de A = 0 à 2, A désignant le nombre d'atomes-grammes de cuivre pour 1 atome-gramme de palladium et (ou) de platine, caractérisé en ce que l'on dépose sur les catalyseurs à régénérer une quantité de cuivre telle que les catalyseurs régénérés possèdent une teneur

**0 056 869**

en cuivre de B = A + C, où A possède la signification définie et C est un nombre valant de 0,5 à 6.

2. Procédé suivant la revendication 1, caractérisé en ce que l'on réactive des catalyseurs sur support avec une teneur en palladium ou en platine de 0,1 à 25% en poids, en tellure de 0,01 à 30% en poids et en cuivre de 0 à 30% en poids.

3. Procédé suivant la revendication 1, caractérisé en ce que l'on réactive des catalyseurs sur support contenant par atome-gramme de palladium ou de platine entre 0,01 et 2 atomes-grammes de cuivre et entre 0,01 et 1 atome-gramme de tellure.

4. Procédé suivant la revendication 1, caractérisé en ce que les catalyseurs sur support comprennent comme matériau support du charbon actif.

5. Procédé suivant la revendication 1, caractérisé en ce que les catalyseurs sur support comprennent comme matériau support du gel de silice (silicagel).

6. Procédé suivant la revendication 1, caractérisé en ce que les catalyseurs sur support sont chauffés entre 400 et 900°C après le dépôt du cuivre.

**Claims**

1. A process for regenerating a supported catalyst for acyloxylations, which contains palladium and/or platinum and tellurium, with or without copper, the copper content A being from 0 to 2, where A is the number of gram atoms of copper per gram atom of palladium and/or platinum, wherein sufficient copper is applied to the catalyst to be regenerated that the regenerated catalyst has a copper content B = A + C, where A has the above meaning and C is a number from 0.5 to 6.

2. A process as claimed in claim 1, wherein a supported catalyst which contains from 0.1 to 25 per cent by weight of palladium or platinum, from 0.01 to 30 per cent by weight of tellurium and from 0 to 30 per cent by weight of copper is reactivated.

3. A process as claimed in claim 1, wherein a supported catalyst which contains from 0.01 to 2 gram atoms of copper and from 0.01 to 1 gram atom of tellurium per gram atom of palladium or platinum is reactivated.

4. A process as claimed in claim 1, wherein the supported catalyst to be reactivated contains active charcoal as the carrier.

5. A process as claimed in claim 1, wherein the supported catalyst to be reactivated contains silica gel as the carrier.

6. A process as claimed in claim 1, wherein the supported catalyst is heated at 400–900°C after the copper has been applied.

Versuchsdauer (Beispiel 4)

Katalysator-Aktivität
(g BEDA pro kg Kat. u. Std.)

Cu — Gehalt des Kat.
(Gew.-%)

500

400

300

200

100

6

10

8

6

4

2

Regenerierung

Katalysator-Aktivität

Cu-Gehalt

0    100    200  3500    3600    3700

Versuchsdauer (h)

0 056 869